# EUROPEAN PATENT SPECIFICATION

(11) **EP 2 866 790 B1**
(45) Date of publication and mention of the grant of the patent: **24.05.2017**
(21) Application number: 13733948.7
(22) Date of filing: 05.06.2013
(51) Int. Cl.: A61K 9/127, A61K 47/64

(54) **LIPOSOMES ACTIVE IN-VIVO ON NEURODEGENERATIVE DISEASES (IN PARTICULAR ALZHEIMER'S DISEASE)**
IN-VIVO AKTIVE LIPOSOMEN BEI NEURODEGENERATIVEN ERKRANKUNGEN (INSBESONDERE MORBUS ALZHEIMER)
LIPOSOMES ACTIFS IN VIVO SUR DES MALADIES NEURODÉGÉNÉRATIVES (EN PARTICULIER LA MALADIE D'ALZHEIMER)

(30) Priority: 28.06.2012 US 201213536851
(43) Date of publication of application: 06.05.2015
(73) Proprietor: Università degli Studi di Milano - Bicocca, 20126 Milano (IT)
(72) Inventor: RE, Francesca, I-20020 Busto Garolfo (MI) (IT); SANCINI, Giulio, I-20060 Gessate (MI) (IT); FORLONI, Gianluigi, I-20017 Rho (MI) (IT); SALMONA, Mario, I-20155 Milano (IT); MASSERINI, Massimo, I-16038 Santa Margherita Ligure (Genova) (IT)
(74) Representative: Gerli, Paolo
(86) International application number: PCT/EP2013/001660
(87) International publication number: WO 2014/000857

(56) References cited:
- FRANCESCA RE ET AL: "Functionalization with ApoE-derived peptides enhances the interaction with brain capillary endothelial cells of nanoliposomes binding amyloid-beta peptide", JOURNAL OF BIOTECHNOLOGY, ELSEVIER SCIENCE PUBLISHERS, AMSTERDAM, NL, vol. 156, no. 4, 29 June 2011 (2011-06-29) , pages 341-346, XP028119269, ISSN: 0168-1656, DOI: 10.1016/J.JBIOTEC.2011.06.037 [retrieved on 2011-07-06]

## Description

### STATE OF THE ART

Alzheimer's disease is a neurodegenerative disease characterized by progressive loss of memory and cognitive function. It is classifiable as dementia and it is likely to be the fourth most common cause of death in industrialized countries, in addition to causing incalculable social and economic harm. The post-mortem observation of brain tissues of Alzheimer's patients reveals the presence of senile plaques and neurofibrillary tangles in the limbic system and cortex. The plaques, which are mainly present in the extracellular level, contain as a major component the β-amyloid peptide with 40 or 42 amino acids; the β-amyloid peptide (herein abbreviated Aß) is produced in significant amounts in the brain of patients in the form of monomer, and tends to associate to form increasingly larger aggregates (oligomers, fibrils, plaques). Aggregates are toxic to neurons, leading to their degeneration responsible for progressive loss of cognitive ability and ultimately death. The peptide Aß, produced in abnormally high amounts in Alzheimer's disease, accumulates in the brain.

So far only modest, unsatisfactory reduction of the plaque have been obtained. For example, the literature reports a reduction of the amyloid plaque by using anti-Aβ antibodies or gangliosides; however these substances, beyond providing a modest plaque reduction, are associated with risks of serious side effects on the immune system, e.g. meningoencephalitis (J.Neurosci.2011, 31(25), 9323-31). The patent application WO 2009/150686 describes liposomes based on cholesterol and sphingomyelin, and a further lipid chosen among cardiolipin, phosphatidic acid and phosphatidylglycerol; these products have a binding capacity with the Aβ peptide *in*-*vitro*; no *in vivo* data are reported, nor studies showing the inhibition of plaque formation.

The article J.Biotechnology 156, 2011, 341-346 describes new liposomes functionalized with phosphatidic acid and a modified apolipoprotein E-derived peptide, showing a capacity of internalization into brain endothelial cells; the liposomes were also found capable to bind the Aβ peptide in an *in*-*vitro* assay, such binding being attributed to the sole phosphatidic acid component of the liposome: in fact the additional presence of apolipoprotein was shown not to improve binding to Aβ peptide, on the contrary it partially hindered it; this publication does not investigate the capability of these liposomes to inhibit the aggregation of Aβ peptide into fibrils and plaques, nor the disgregation of aggregates.

In another publication (Nanomedicine: Nanotechnology, Biology and Medicine, 7(2011), 541-550), liposomes functionalized with phosphatidic acid were investigated for their practical capability to inhibit the aggregation of the peptide Aß into fibrils, i.e. to prevent the plaque-forming process: the obtained data showed the failure of liposomes functionalized with phosphatidic acid to inhibit such aggregation.

In summary, although some examples of Aβ peptide-binding liposomes are known, at present there is no evidence that any such products can bring about a concrete and significant reduction of the amyloid plaque in vivo. On the contrary, although some theoretical models of activity were hypothesized for certain functionalized liposomes, no evidence of inhibition of the pathologic process of Aß amyloid plaque formation has ever been reported, and the available evidences suggest, e.g. for phosphatidic acid-based liposomes, a lack of effectiveness to inhibit the aggregation of Aß peptide into senile plaques.

Moreover, in the field of liposomes containing added substituents or functional arms ("functionalized liposomes"), there is no rationale or evidence for constructing liposomes in which two or more added substituents can synergize, at the same biochemical level, in reducing plaque formation.

The need remains therefore highly felt for agents capable to maximize the inhibition of the Aß peptide aggregations into fibrils and plaques *in vivo*, so as to concretely inhibit a main pathological component of Alzheimer's disease. The need is also felt for treatments being strongly effective against the amyloid plaque *in*- *vivo*, avoiding as far as possible using extraneous or agents suspected of toxic side effects (gangliosides, etc.) so as to represent a safe treatment of Alzheimer's disease and other neurodegenerative conditions.

Moreover, although some theoretical mechanisms involved with aggregation of the Aß peptide plaque have been proposed, there is no evidence so far that the inhibition of any such mechanisms is sufficient to treat Alzheimer's disease in affected patients or animal models thereof. The need is thus felts for drugs which can bring about a real and consistent improvement in the established behavioural damages of Alzheimer's disease, such as loss of cognitive abilities, memory loss and dementia.

### SUMMARY OF THE INVENTION

The present invention concerns liposomes comprising: (i) phosphatidic acid and/or cardiolipin; (ii) apolipoprotein E (ApoE) for use in treating or preventing Alzheimer's disease, reducing or eliminating the amyloid plaque in the central nervous system of the patient at both intracellular and extracellular level. The so modified liposomes, administered in an animal model of Alzheimer's disease, obtained a dramatic *in-vivo* reduction of the amyloid plaque, obtained via a synergistic interaction of its components. This is a first example of a strong therapeutic response to Aβ peptide binding strategies, obtained *in-vivo* at moderate doses, using agents having low toxicity risks, allowing an effective treatment of neurodegenerative diseases, in particular Alzheimer's disease.

### DESCRIPTION OF THE DRAWINGS

Figure 1: Treatment of APP-swe transgenic mice with double-functionalized liposomes. mApo = mice treated with Liposomes Functionalized with modified ApoE peptide, without phosphatidic acid; PA-mApo = mice treated with Liposomes Functionalized with phosphatidic acid and with modified ApoE peptide.
Figure 2: Content of Aβ 40 or 42 in brain homogenates of APP-swe transgenic mice treated with liposomes functionalized with modified ApoE peptide (mApo) or modified ApoE peptide + phosphatidic acid (PA-mApo).
Figure 3: PA-mApo liposomes inhibit Aβ42 aggregation (A) and stimulate disaggregation of pre-formed aggregates (B) in vitro.
Figure 4A Systemic administration of PA-Mapo liposomes reduce Aβ42 brain levels in AD double TG mice.
Fibure 4B: Systemic administration of PA_Mapo-liposomes induced a significant reduction in the brain Aβ plaques of double TG mice compared to TG mice treated with phosphate buffered saline or with PA-liposomes.
Figure 4C. PA-mApo liposomes restore cognitive deficit in AD double TG mice, assessed by Object recognition test

### DETAILED DESCRIPTION OF THE INVENTION

The term "phosphatidic acid" used herein means a molecule having a glycerol backbone esterified with two fatty acids and one phosphate group, for example dimiristoylphosphatidic acid. Cardiolipin is a well-known compound, also known as 1,3-bis(sn-3'-phosphatidyl)-sn-glycerol. In the liposome, the phosphatidic acid (or cardiolipin) is preferably present in 1-20 %, more preferably 1-10%, most preferably 5% molar amount (with respect to the total moles of all substances making up the liposome). If phosphatidic acid or cardiolipin are both present, the above ranges are referred to the sum of moles of both substances; when both present, phosphatidic acid and cardiolipin can be used in any mutual ratio.

Apolipoprotein E (ApoE) is a 34 kDa glycoprotein containing 299 aminoacids, produced in high levels in liver and brain. ApoE can be used as obtained from its natural sources or be synthetized on purpose. The term "ApoE" includes the known ApoE isoforms, coded E2, E3. The term "ApoE" also includes fragments of ApoE; preferably those belonging to the aminoacid sequence 100-200, more preferably 120-170; a preferred fragment is the one consisting in the aminoacids 141-150 of ApoE (Sequence: (LRKLRKRLLR)-NH₂) or dimer thereof (Sequence: (LRKLRKRLLR)-(LRKLRKRLLR)-NH₂). Preferably, the ApoE or fragment thereof includes a terminal, cystein-ending, small peptide sequence (up to five aminoacids), such as the tripeptide CWG-, assisting in the chemical linkage to the lipid: preferred examples of the resulting fragments are: CWG-(LRKLRKRLLR)-NH₂ , herein "mApoE" , or CWG-(LRKLRKRLLR)-(LRKLRKRLLR)-NH₂ , herein "dApoE".

Said ApoE may be present in the liposomes as a physical mixture with the other lipids constituents of the liposome membrane, or be deposited on such membrane, or be chemically linked to these lipids, or be contained within the liposome, or be added to existing liposomes, etc. Preferably, it is chemically linked. In this case, the ApoE as above described may be linked or connected via a linker molecule; a preferred linker is the compound 1,2 stearoyl-sn-glycero-3-phosphoethanolamine-N-[maleimide(poly(ethylene glycol)-2000)] (herein abbreviated "mal-PEG-PE"). In the final liposome, the ApoE is preferably present in a molar amount of 1-5 % (with respect to the total moles of all substances making up the liposome).

The final claimed liposome further comprises standard liposome lipids. These make up the bulk of the liposome, preferably accounting for a 90-98% molar amount (with respect to the total moles of all substances making up the liposome). Preferred standard liposome lipids are sphingomyelin, phosphatidylcholine, phosphatidylethanolamine (PEGylated or not) and cholesterol.

The above described liposomes are per se fully effective on the amyloid plaque *in-vivo*, as herein demonstrated; as an option, they may additionally include further active agents, also useful for the intended treatment; in such case the present liposomes perform the double function of drug and drug carrier. The preparation process for the claimed liposomes can be any known method to prepare liposomes, characterized by the use of the components (i), (ii) as described above. Exemplary known methods are: extrusion, lipid hydration, solvent spherule method, sonication, French press cell, solvent injection, detergent removal, reserve phase evaporation, calcium-induced fusion, microfluidization, freeze-thawing, freeze-drying, etc. The process further involves the addition of the standard liposome lipids described above, which make up the bulk of the liposome.

The ApoE-adding step, typical of the present invention, is performed in function of the desired mode of connection with ApoE. For example, if ApoE is added as a physical mixture with the other lipids, a suitable procedure may involve:
(a) mixing in a solvent: the components (i) and standard liposome lipids;
(b) drying the mixture of step (a);
(c) rehydrating the mixture of step (b) in a buffer solution;
(d) stirring and then extruding the solution of step (c) through a filter with suitable pore size, e.g. 100 nm;
(e) add component (ii);

If ApoE is added via a linker molecule, a suitable procedure may involve:
(a) mixing in a solvent: the components (i), the linker molecule and standard liposome lipids
(b) drying the mixture of step (a);
(c) rehydrating the mixture of step (b) in a buffer solution;
(d) stirring and then extruding the solution of step (c) through a filter having suitable pore size, e.g. 100 nm;
(e) purifying the extruded solution of step (d);
(f) resuspending in a buffer the purified solution of step (e) and mixing it with the component (ii);
(g) purifying the solution of step (f), and final drying.

A further object of the invention are pharmaceutical compositions comprising the above described and claimed liposomes, together with one or more pharmaceutically acceptable excipients and, optionally, further active agents. The type and amounts of excipients are chosen in function of the chosen pharmaceutical form; suitable pharmaceutical forms are liquid systems like solutions, infusions, suspensions; semisolid systems like colloids, gels, pastes or creams; solid systems like powders, granulates, tablets, capsules, pellets, microgranulates, minitablets, microcapsules, micropellets, suppositories; etc. Each of the above systems can be suitably be formulated for normal, delayed or accelerated release, using techniques well-known in the art.

A further object of the invention are the above described liposomes or pharmaceutical compositions for use in a method to treat or prevent particular Alzheimer's diseases

In the experimental section of this application, the efficacy of the present treatment has been documented by histological and behavioral findings obtained by the Applicant on an animal model of Alzheimer's Disease.

The claimed liposome are especially active in inhibiting (i.e. preventing, reducing or eliminating; eliminating being preferred) *in*-*vivo* the amyloid plaque in the central nervous system. This activity is useful in patients suffering from or being at risk of developing the above diseases; as an additional advantage, said plaque inhibition takes place in the CNS both at extracellular and intracellular level, thus extending the activity to all Aβ deposits present in the central nervous system: this is an essential prerequisite to achieve a total elimination of the plaque.

The invention thus includes medical uses aimed at inhibiting *in*-*vivo* the amyloid plaque in the central nervous system of patients suffering from or being at risk of developing the above diseases, characterized by the administration of the above described liposomes or pharmaceutical compositions.

Further described are *in vitro* methods to provide an enhanced reduction of Aβ deposits, characterized by including ApoE or a derivative thereof in a liposome containing phosphatidic acid and/or cardiolipin, taking advantage from the unexpected finding that these components cooperate synergically in inhibiting the aggregation of Aβ peptide into fibrils and plaques and stimulate disaggregation of pre-existing aggregates.

The above treatments can be performed by delivering the above liposomes / pharmaceutical compositions to a patient in need thereof, in suitable dose unit, via any suitable administration route.

Suitable dose units are comprised in the 1-15 mmoles total lipids for an average 70 kg patient. All administration routes (enteral, parenteral) enabling a systemic distribution of the medicament are contemplated. Example of possible administration routes are: oral, intravenous, intramuscular, inhalatory, intratracheal, intraperitoneal, buccal, sublingual, nasal, subcutaneous, transdermal, transmucosal. In the present invention, the administration routes directly into the central nervous system (i.e. into those areas placed beyond the blood brain barrier) are definitely not necessary for the present liposomes, since they are advantageously active in the CNS via simple systemic administration. From the therapeutic perspective it is primarily important that the treatment with the present liposomes obtains a strong *in*-*vivo* reduction of the amyloid plaque via systemic administration, by means of a low-toxicity active agent, exempt from side effects, at moderate dosages. A long-wanted *in-vivo* effective treatment is thus provided, easy-to-perform, involving a non-expensive process of preparation, useful for the treatment and prevention of diseases depending from the formation of amyloid plaque, in particular neurodegeneration in the central nervous system, more particularly Alzheimer's Disease and related dementias. The invention is now further disclosed by means of the following non-limitative examples.

### EXPERIMENTAL PART

### 1. PREPARATION OF LIPOSOMES

### Liposomes composition:

5 mol% phosphatidic acid
2.5 mol% PEG-PE-maleimide (mal-PEG-PE) assisting in the chemical linkage to the ApoE:
   46.25 mol% cholesterol
   46.25 mol% sphingomyelin

The liposomes were prepared by extrusion technique. The lipids were mixed in an organic solvent (chloroform / methanol 2:1, v:v), which was subsequently removed by a gentle flow of nitrogen, followed by vacuum pump for at least 3 hours. The so obtained lipid film was resuspended in physiological buffer (150 mM NaCl, pH 7.40) and extruded 10 times under pressure (20 bar) at a temperature of 55 ° C through polycarbonate filters with pore diameter of 100 nm using an Extruder (Lipex Biomembranes).

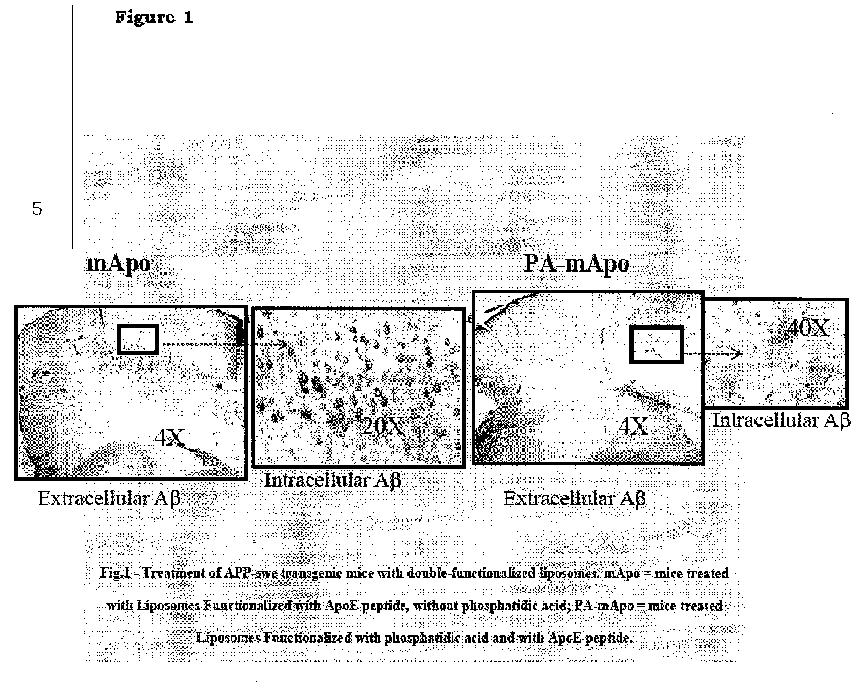

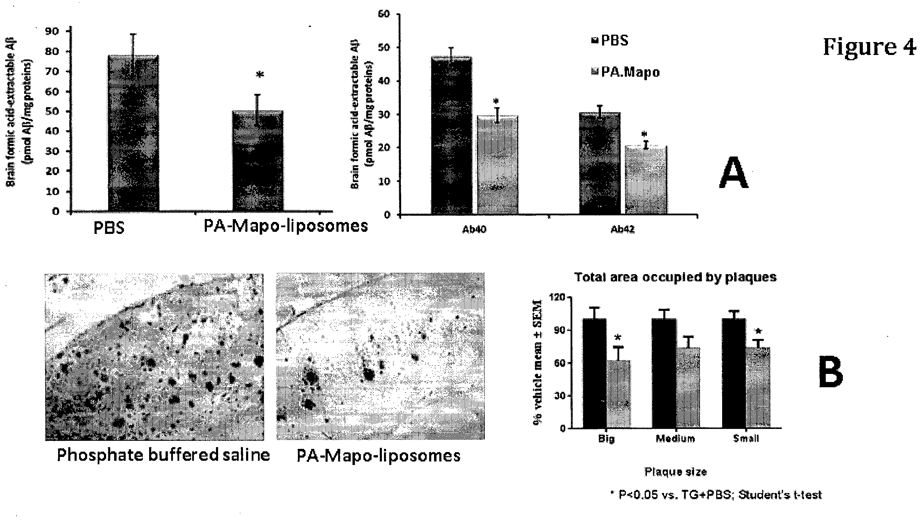

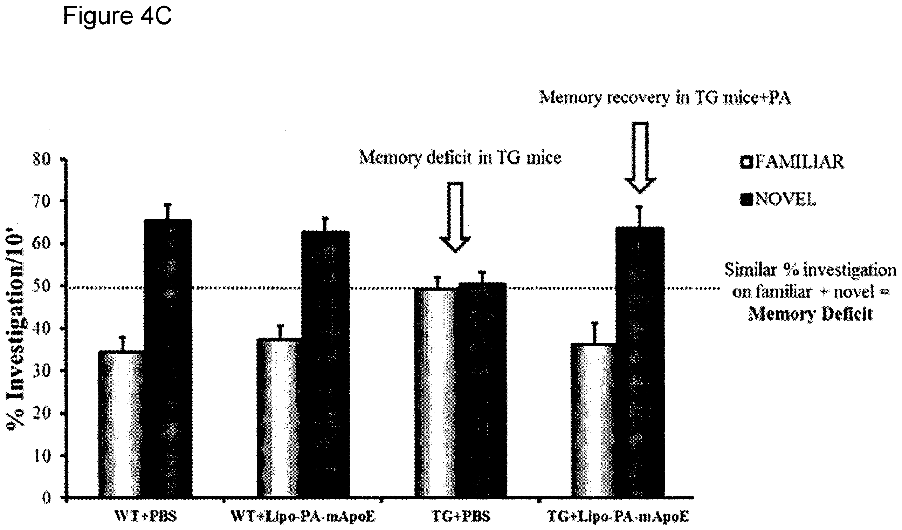

The mApoE peptide was added to the liposomes in the molar ratio of 1.2:1 (peptide: mal-PEG-PE) and the mixture was incubated overnight at room temperature. The next day the mixture was injected intratracheally (i.t.) (Fig. 1-2) or intraperitoneally (Fig.4) into healthy mice or in transgenic mice for Alzheimer's disease (for tests on amyloid plaque)

### 2. ANIMAL MODELS

- Balb/C healthy mice
- C56 mice transgenic for the mutant APP-swe.
- C56 mice transgenic for the APP-PS1 double mutation

### 3. PHARMACOKINETIC TESTS

A group of healthy mice received i.t. administered liposomes containing phosphatidic acid and mApoE and comprising radioactive (3H-SM) sphingomyelin and (14C-PA) phosphatidic acid, following this treatment schedule: 1 injection every 2 days for 5 days of 100uL aliquot (40 mM total lipid) of a liposomes. The animals were sacrificed at various times after administration. The organs were taken and processed for measuring radioactivity through liquid scintillation.

The results reported below refer to the measurements of radioactivity in the brain, reported as % of total dose administered. The radioactivity in the brain after the 3^{rd} administration indicated a presence of 0.7% of the administered dose, corresponding to substantially very low amount reaching the brain.

### 4. TESTS ON AMYLOID PLAQUE IN-VIVO

A first group of APP-swe mice (genetically modified for Alzheimer's disease) was administered with liposomes containing phosphatidic acid, and mApoE (group "PA-Mapo", n = 6), a second group of mice APP-swe received the same liposomes but without phosphatidic acid (Group "Mapo", n = 6). In both cases, the treatment consisted of 3 i.t. injections per week for 3 weeks, 100µL of a 40mM liposome suspension per injection. The mice were finally sacrificed and the brain removed. Cryo-sections were prepared for subsequent histological examination and homogenates for ELISA. The histological analysis was performed on cryo-sections of the brain using anti-Aß antibodies and subsequent staining with secondary antibodies HRP/chromogen. Staining (brown) corresponds to Aß deposits.

### Histological examination results

As can be seen in Fig. 1 (images before and after treatment in the group "PA-Mapo"), the treatment with the liposomes of the invention has removed the deposits of Aß; removal was found effective at both extra and intra-cellular level. In particular, the total elimination of deposits was observed in 4 out of 6 treated animals. Conversely in the "Mapo" group, no removal of Aß deposits was observed.

This confirms the efficacy of the method in accordance with the invention. In particular it can be deduced that the association mApoE+PA allows to obtain the vivo reduction/elimination of Aβ peptide deposits.

### ELISA Results

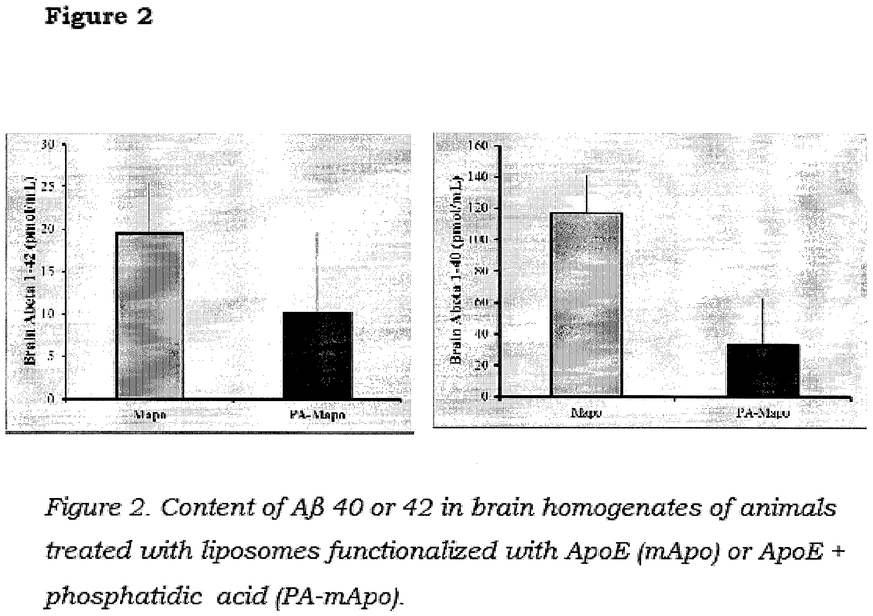

After extraction with formic acid from brain tissue, the total content of beta-amyloid (1-40 and 1-42) was quantified by ELISA assay. As can be seen from Figure 2, it is noted a significant decrease of the total content of the two forms of Aβ in the PA-Mapo group, compared to the mApo group.

### 5. ADDITIONAL TESTS ON AMYLOID PLAQUE INHIBITION IN VITRO

The liposomes effect on Aβ42 aggregation has been evaluated by Thioflavin T (ThT) assay by monitoring the peptide aggregation over the time in the presence of different amount of liposomes in comparison to the peptide alone in solution. Samples enriched of monomeric Aβ42 (25 µM) have been incubated for different times (up to 120 h) in the presence of different liposomes at different concentrations. After different intervals of time, aliquots have been analysed after addition of ThT and by measuring the fluorescence. An increase of fluorescence intensity indicates an increase in the Aβ42 aggregation.

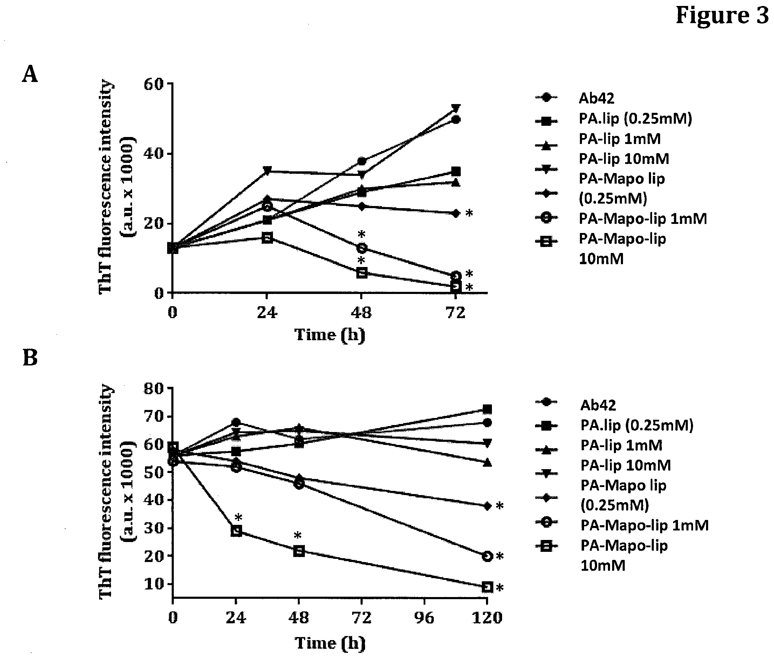

The results (FIG.3A) showed that the presence of PA-Mapo-liposomes are strongly more potent that PA-liposomes in inhibiting the Aβ aggregation process. In this test mApoE synergistically cooperated with phosphatidic acid in inhibiting the Aβ plaque formation process.

The effect of liposomes on disaggregation on pre-formed Aβ aggregates (25 µM) was also investigated by ThT assay in vitro. Aβ42 fibrils have been prepared by incubation of Aβ monomers for 2h at 37°C in HCl 10mM and then incubated with different doses of PA-Mapo-liposome. The results (FIG.3B) showed that the presence of PA-Mapo-liposomes in the mixture induced a dose- and time-dependent decrease of the initial fluorescence of Aβ fibrils, showing their disaggregation features. On the contrary, PA-liposomes did not shown any significant effect on the Aβ fibrils.

### 6. ADDITIONAL TESTS ON AMYLOID PLAQUE INHIBITION IN VIVO

PA-MApo-liposomes (40mM total lipids concentration, 100 uL volume of injection) have been administered via i.p. injection to animal models of AD (APP/PS1 2Tg mice). Mice were chronically treated with 3 injections/week for 3 weeks. Subsequently, mice were sacrificed and the plasma, brain, liver and spleen were dissected and analyzed for the content of Aβ 40 and 42 by ELISA assay after tissue homogenization and Aβ peptide extraction. Moreover, the brain was also analyzed for the Aβ content by immunohistochemical procedure.

The results (FIG.4A) showed that the treatment with PA_Mapo-liposomes induced an average 40 % of reduction of the total Aβ brain content, compared to the peptide amount in control mice (TG mice treated with phosphate buffered saline). This reduction was measured for both peptide fragment Aβ 40 and 42 and also for both soluble and insoluble Aβ aggregates.

The immunohistochemical analysis (FIG.4B) of brain slices, shows that the treatment with PA_Mapo-liposomes induced about 35% reduction in the brain Aβ plaques of TG mice compared to TG mice treated with phosphate buffered saline. Similar experiments carried out with PA-liposomes or Mapo-liposomes did not show any effect on aggregation of brain Aβ. This confirms the fundamental contribution of the functionalization with ApoE in inhibiting the Aβ peptide aggregation.

### 7. BEHAVIOURAL EFFECTS ON ALZHEIMER's DISEASE ANIMAL MODEL

The same mice undergoing the experiment described above in example 6, at the end of treatment with liposomes and before sacrification, were subjected to a behavioral test (object recognition test) to assess either the potential toxicity of liposomes (experiments carried out on healthy mice of the same age) and the possible recovery of the memory deficit in TG mice)

Mice were exposed to two identical objects for 10 min/day for 3 days. After training phase, one of the two objects was changed with a new one of different color and shape. The time spent exploring each of the objects was quantified and expressed as % of investigation in 10 min.

The results, shown in Fig. 4C are the following:
HEALTHY MICE (WT): healthy mice spent more time to explore the new object because they remember the older one.
AD MICE: TG mice spent almost the same time around the two objects because they don't remember the older one.

AD MICE treated with PA-Mapo. The behavioral test shown that the treatment of TG D mice with PA-Mapo-liposomes restore the memory deficit and did not induce any changes in the learning and memory of healthy mice.

## Claims

1. Liposomes comprising: (i) phosphatidic acid and/or cardiolipin; (ii) apolipoprotein E, for use in treating or preventing Alzheimer's disease, reducing or eliminating the amyloid plaque in the central nervous system of the patient at both intracellular and extracellular level.

2. Liposomes for use according to claim 1, wherein the component (i) is present in 1-20% molar percentage.

3. Liposomes for use according to claim 1, wherein the component (i) is present in 1-10% molar amount.

4. Liposomes for use according to claim 1, wherein the component (i) is present in 5% molar amount.

5. Liposomes for use according to claims 1-4, wherein the component (ii) is present in 1-5% molar amount.

6. Liposomes for use according to claims 1-5, wherein the component (ii) is an ApoE fragment.

7. Liposomes for use according to claim 6, wherein the ApoE fragment is chosen within the aminoacid sequence 100-200 of ApoE.

8. Liposomes for use according to claim 6, wherein the ApoE fragment is chosen within the aminoacid sequence 120-170 of ApoE.

9. Liposomes for use according to claim 6, wherein the ApoE fragment corresponds to the sequence 141-150 or a dimer thereof.

10. Liposomes for use according to claims 1-9 , wherein said apolipoprotein E includes, at its C-terminal, a cystein-ending tripeptide.

11. Liposomes for use according to claim 10, wherein the cystein-ending tripeptide is CWG-.

12. Liposomes for use according to claims 1-11, wherein the component (ii) is chemically linked via a linker molecule, said linker molecule being 1,2 stearoyl-sn-glycero-3-phosphoethanolamine-N-[maleimide(poly(ethylene glycol)-2000)].

13. Liposomes for use according to claims 1-12, including further standard liposome lipids selected from one or more among sphingomyelin, phosphatidylcholine, phosphatidylethanolamine (PEGylated or not) and cholesterol.

14. Liposomes for use according to claim 13, wherein the standard liposomes lipids are cholesterol and sphingomyelin, present in equimolar ratio.

15. Pharmaceutical composition containing the liposomes as described in claims 1-14 and pharmaceutically acceptable excipients.

16. Pharmaceutical compostion according to claim 15, in the form of a dose unit suitable to administer from 1 to 15 mmoles of total lipids to a 70 Kg patient.

17. Pharmaceutical composition according to claim 16, formulated for a systemic administration route.

## Patentansprüche

1. Liposome enthaltend: (i) Phosphatidsäure und/oder Cardiolipin; (ii) Apolipoprotein E zur Verwendung bei der Behandlung von oder der Vorbeugung gegen Alzheimer-Krankheit, zum Reduzieren oder Eliminieren amyloider Plaques im zentralen Nervensystem des Patienten auf sowohl intrazellulärer als auch extrazellulärer Ebene.

2. Liposome zur Verwendung gemäß Anspruch 1, wobei die Komponente (i) in molarem Anteil von 1 bis 20% vorhanden ist.

3. Liposome zur Verwendung gemäß Anspruch 1, wobei die Komponente (i) in molarer Menge von 1 bis 10% vorhanden ist.

4. Liposome zur Verwendung gemäß Anspruch 1, wobei die Komponente (i) in molarer Menge von 5% vorhanden ist.

5. Liposome zur Verwendung gemäß den Ansprüchen 1 bis 4, wobei die Komponente (ii) in molarer Menge von 1 bis 5% vorhanden ist.

6. Liposome zur Verwendung gemäß den Ansprüchen 1 bis 5, wobei die Komponente (ii) ein ApoE-Fragment ist.

7. Liposome zur Verwendung gemäß Anspruch 6, wobei das ApoE-Fragment aus der Aminosäure-Sequenz 100 bis 200 des ApoE ausgewählt ist.

8. Liposome zur Verwendung gemäß Anspruch 6, wobei das ApoE-Fragment aus der Aminosäure-Sequenz 120 bis 170 des ApoE ausgewählt ist.

9. Liposome zur Verwendung gemäß Anspruch 6, wobei das ApoE-Fragment der Sequenz 141 bis 150 oder einem Dimer davon entspricht.

10. Liposome zur Verwendung gemäß der Ansprüche 1 bis 9, wobei das Apolipoprotein E an seinem C-Terminus ein Cystein-endendes Tripeptid enthält.

11. Liposome zur Verwendung gemäß Anspruch 10, wobei das Cystein-endende Tripeptid CWG- ist.

12. Liposome zur Verwendung gemäß der Ansprüche 1 bis 11, wobei die Komponente (ii) chemisch verbunden ist über ein Linker-Molekül, wobei das Linker-Molekül 1,2-Stearoyl-sn-glycero-3-phosphoethanolamin-N-[maleimid(poly(ethylenglykol)-2000)] ist.

13. Liposome zur Verwendung gemäß der Ansprüche 1 bis 12, weiter beinhaltend Standard-Liposom-Lipide ausgewählt von einem oder mehreren aus Sphingomyelin, Phosphatidylcholin, Phosphatidylethanolamin (PEGyliert oder nicht) und Cholesterol.

14. Liposome zur Verwendung gemäß Anspruch 13, wobei die Standard-Liposom-Lipide Cholesterol und Sphingomyelin sind, vorhanden in äquimolarem Verhältnis.

15. Pharmazeutische Zusammensetzung beinhaltend die Liposome, wie in den Ansprüchen 1 bis 14 beschrieben und pharmazeutisch akzeptable Hilfsstoffe.

16. Pharmazeutische Zusammensetzung gemäß Anspruch 15, in Form einer Dosiereinheit geeignet, um einem 70 kg Patienten 1 bis 15 mmol Gesamtlipide zu verabreichen.

17. Pharmazeutische Zusammensetzung gemäß Anspruch 16, entworfen für eine systemische Verabreichungsweg

## Revendications

1. Liposomes comprenant : (i) de l'acide phosphatidique et/ou de la cardiolipine ; (ii) de l'apolipoprotéine E, pour utilisation dans le traitement ou la prévention de la maladie d'Alzheimer, la réduction ou l'élimination de la plaque amyloïde dans le système nerveux central du patient tant au niveau intracellulaire qu'au niveau extracellulaire.

2. Liposomes pour utilisation selon la revendication 1, dans lesquels le composant (i) est présent en un pourcentage molaire de 1 à 20 %.

3. Liposomes pour utilisation selon la revendication 1, dans lesquels le composant (i) est présent en une quantité molaire de 1 à 10 %.

4. Liposomes pour utilisation selon la revendication 1, dans lesquels le composant (i) est présent en une quantité molaire de 5 %.

5. Liposomes pour utilisation selon les revendications 1 à 4, dans lesquels le composant (ii) est présent en une quantité molaire de 1 à 5 %.

6. Liposomes pour utilisation selon les revendications 1 à 5, dans lesquels le composant (ii) est un fragment d'ApoE.

7. Liposomes pour utilisation selon la revendication 6, dans lesquels le fragment d'ApoE est choisi parmi la séquence d'acides aminés 100-200 de l'ApoE.

8. Liposomes pour utilisation selon la revendication 6, dans lesquels le fragment d'ApoE est choisi parmi la séquence d'acides aminés 120-170 de l'ApoE.

9. Liposomes pour utilisation selon la revendication 6, dans lesquels le fragment d'ApoE correspond à la séquence 141-150 ou à un dimère de celle-ci.

10. Liposomes pour utilisation selon les revendications 1 à 9, dans lesquels ladite apolipoprotéine E inclut, au niveau de sa terminaison C, un tripeptide à terminaison cystéine.

11. Liposomes pour utilisation selon la revendication 10, dans lesquels le tripeptide à terminaison cystéine est le CWG-.

12. Liposomes pour utilisation selon les revendications 1 à 11, dans lesquels le composant (ii) est lié chimiquement via une molécule de liaison, ladite molécule de liaison étant le 1,2-stéaroyl-sn-glycéro-3-phosphoéthanolamine-N-[maléimide(poly(éthylène glycol)-2000)].

13. Liposomes pour utilisation selon les revendications 1 à 12, incluant d'autres lipides liposomaux standards choisis parmi un ou plusieurs composés de sphingomyéline, de phosphatidylcholine, de phosphatidyléthanolamine (PEGylée ou non) et de cholestérol.

14. Liposomes pour utilisation selon la revendication 13, dans lesquels les lipides liposomaux standards sont le cholestérol et la sphingomyéline, présents dans un rapport équimolaire.

15. Composition pharmaceutique contenant les liposomes tels que décrits dans les revendications 1 à 14 et des excipients pharmaceutiquement acceptables.

16. Composition pharmaceutique selon la revendication 15, sous la forme d'une unité de dose adaptée à l'administration de 1 à 15 mmoles de lipides totaux à un patient de 70 kg.

17. Composition pharmaceutique selon la revendication 16, formulée pour une voie d'administration systémique.
